# EUROPEAN PATENT APPLICATION

(11) **EP 1 020 520 A1**
(43) Date of publication of application: **19.07.2000**
(21) Application number: 99200119.8
(22) Date of filing: 15.01.1999
(51) Int. Cl.: C12N 15/12, C12N 15/86, A61K 48/00, C12N 5/10, A61K 38/17, C07K 14/82

(54) **Prevention of immune related removal of cells from the mammalian body, mutant PML molecules useful therefor**

(71) Applicant: Introgene B.V., 2333 AL Leiden (NL)
(72) Inventor: The designation of the inventor has not yet been filed
(74) Representative: Ottevangers, Sietse Ulbe

(57) **Abstract**

The present invention provides means and methods for at least in part inhibiting an immune response against specific cells in a body without essentially affecting the capacity of the immune system in said body to mount other immune responses.

## Description

The present invention relates to the field of immune rejection. More in particular the invention lies in the field of prevention of immune related removal of cells from a mammalian body.

### BACKGROUND OF THE INVENTION

Immune related removal of cells from the body is a crucial natural process in the prevention and cure of diseases. However, in current medicine, there is sometimes a need to prevent the activity of the natural system for instance in transplantation. When for instance a kidney is transplanted, the immune system of the recipient often rejects the kidney. Current methods to prevent rejection include the administration of immune suppressing compounds such as cyclosporin A. Such compounds however, have a general immune suppressing activity which also affects the capacity of the immune system to respond to other antigens and thus affects the capacity of the host to mount immune responses against diseases. Preferably one would like to be able to selectively prevent the activity of the immune system toward for instance the transplanted kidney. Also in other applications such as gene therapy and the treatment of auto-immune diseases there is a need to prevent the immune system to act against certain cells while leaving the capacity of the immune system to respond to other cells or other antigens intact.

### Viral and non-viral vectors for human gene therapy

The development of human gene therapy for the treatment of inherited and acquired disorders, requires gene transfer vectors capable of safe and effective delivery and expression of therapeutic genes into human cells. The actual method to introduce and express the genetic material into the target cells of a patient is a key component in every gene therapy protocol. Several different gene transfer systems are currently employed to insert therapeutic genes into somatic cells. These methods can be divided in viral and non-viral gene delivery methods.

Non-viral systems for nucleic acid delivery into target cells include a variety of nucleic acid-mediated methods, like direct injection of naked nucleic acid and particle bombardment. To overcome the limitations of these nucleic acid-based delivery methods (low transfer efficiency, and cell toxicity), alternative systems have been developed utilising the entrapment of the nucleic acid in vesicles (like liposomes), or binding of the nucleic acid to synthetic conjugates (like e.g. transferrin-polylysine). Such conjugate-nucleic acid complexes can be delivered via receptor mediated endocytosis.

Viral gene transfer systems are based on the natural capability of viruses to deliver their genes in mammalian cells. The high gene-transfer efficiency of viruses has led to the development of viral vectors in which part of the viral genome is replaced by a transgene to be introduced into eukaryotic cells. The most commonly used viral systems are based on retroviruses, adenoviruses and adeno-associated viruses. Retroviruses and adeno-associated viruses have been a focus for development because of their capacity to stably integrate DNA sequences in target cells.

Each of the above-mentioned viral and non-viral delivery systems has its own characteristics in terms of efficiency of gene delivery, integration capability, maximum insert size of the recombinant gene, vector yields, stability of expression, etc. Such characteristics may determine which is the most suitable delivery method for a specific gene therapy protocol (reviewed in 1).

However, one feature common to all gene transfer methods is the inherent presentation of antigenic material to the target cell, both by the vector or vehicle, and by the transgene and the encoded gene-product. This presentation may elicit a response of the immune system to the agent that is being delivered into the cells. Immune responses directed against the gene therapy agent and against the transduced cells can lead to destruction of target cells, inflammation and difficulties with vector re-administration. As a consequence, the clinical application of gene therapy vectors is impeded by the potent host immune response to the vector, which limits the duration of its effects.
Immune effectors have been identified as cytotoxic T lymphocytes (CTLs), which destroy vector-transduced cells, as well as B cells, which secrete neutralising antibodies, and block repeated gene transfer.

### The immune system

Cytotoxic T lymphocytes (CTL) continuously monitor the cells and tissues of the body in search of cells synthesising foreign or abnormal proteins. The recognition of abnormal cells (i.e. virus infected) by CTLs requires fragments (peptides) of foreign antigens to be presented at the cell surface of the infected cells in association with class I molecules of the major histocompatibility complex (MHC). Peptides bound by MHC class I molecules have a typical size of 8-12 amino acids with their NH2 and COOH termini anchored in pockets contributing significantly to the stability of the MHC-peptide complex.

The majority of those peptides are generated in the cytosol of virus infected cells from the degradation of polyubiquitinated viral proteins targeted by the 19S proteasome regulator to the multi-catalytic protease complex, the 20S proteasome. In response to the anti-viral cytokine, interferon gamma (IFN- γ) three proteasome subunits, designated LMP2, LMP7 [for latent infection membrane proteins 1 and 2] and MECL1 encoded by genes within the MHC locus, are induced and replace the homologous constitutive proteasome subunits in the 20S mammalian proteasome. The resulting viral peptides can be transported from the cytosol to the endoplasmatic reticulum (ER), through the action of the ATP dependent transporter for antigen presentation (TAP) complex. The TAP complex is composed of two integral ER membrane proteins, encoded by two genes TAP-1 and TAP-2 also located within the MHC locus. MHC class I heavy chains assemble with β2 microglobulin and peptide in the ER to stable heterotrimeric MHC class I complexes, a process which is assisted by transient interactions with chaperon molecules like calnexin, calreticulin and tapasin. Transport of these complexes via the secretory route and their expression at the cell surface enables CTL to play their decisive role in anti viral defence. Yet, several viruses have evolved strategies to evade immune recognition by CTL's by down regulating MHC class I molecules at virtually every step in the antigen presentation pathway (reviewed in 2-5).

Several viruses (i.e. HIV, HBV and EBV) escape from recognition by CTL by producing mutant sequence variants of nominal peptides, which are no longer recognised by the existing repertoire of T cell receptors (6-8). Some other viruses show real intervention with the proteasomal degradation of viral proteins like the P65 and EBNA-1 proteins of human cytomegalovirus (HCMV) and Epstein Barr virus (EBV), respectively (9,10).

The ICP47 protein of Herpes simplex virus type 1 (HSV-1) and HSV-2 each bind to TAP, thereby preventing peptide to associate with TAP at the cytosolic side and the US6 protein of HCMV likewise prevents translocation of peptide, but does so by binding TAP at its ER luminal side (11-14). Some viruses prevent transport of correctly assembled MHC class I-peptide complexes. The E3/19K protein of group C adenoviruses, by direct interaction with MHC class I molecules blocks their egress from ER to the Golgi through an ER retention signal in its cytoplasmic tail (15). Retention of MHC class I peptide complexes in ER is also caused by the US3 product of HCMV, whereas the m152 gene product (gp40) of mouse CMV (MCMV) retains MHC class I complexes in the cis-Golgi compartment independent from its cytoplasmic tail (16-18). By a process that is the reverse of translocation, the US2 and US11 products of HCMV redirect MHC class I heavy chains to the cytosol for degradation by the proteasome (19). The gp48 of MCMV binds to MHC class I complexes, which they route to endosomal/lysosomal compartments by a specific targeting signal (20). The Vpu product of HIV-1 also affects class I expression before export of the molecule from the ER by as yet undefined mechanism (21). Viral strategies to down regulate MHC class I expression even extend to molecules that have reached the cell surface. The Nef protein of HIV-1, by affecting the endocytotic machinery, down-modulates class I molecules from the cell surface, allowing HIV-infected cells to escape killing by CTL (22-24). The gp34 product of MCMV is the fellow traveller of certain MHC class I peptide complexes on their route to the cell surface, where they may function in blocking CTL recognition (25).

### Adenoviruses and immunogenicity

Adenoviruses are convenient viruses for construction of vectors for gene therapy, because of their high efficacy to deliver DNA in most mammalian cell types. Based on the detailed knowledge of their DNA genomes, especially of adenovirus types 2 and 5, recombinant adenoviral vectors have been developed.
Current E1-deleted adenoviral vectors have limited potential in gene therapy because of acute inflammation, resulting in diminished, and transient expression of the transgene.

One of the additional problems associated with the use of recombinant adenoviral vectors is the host-defence reaction against treated cells.
Briefly, first generation recombinant adenoviral vectors are deleted for the E1 region only. The adenoviral E1 products trigger the transcription of the other early genes (E2 - E4), which consequently activate expression of the late viral genes. Therefore, it was generally assumed that E1 deleted vectors would not express any other adenovirus genes. However, many research groups have demonstrated that even in the absence of E1 proteins, residual expression of early and late adenoviral genes occurs.
In a gene therapy setting, this means that transfer of the therapeutic recombinant gene to somatic cells not only results in expression of the therapeutic protein but also in the synthesis of viral proteins. Both CD4 (T-helper) and CD8 (Cytotoxic T lymphocytes) are involved in the immune response against adenovirus infected cells. Cells that express adenoviral proteins are recognised and killed by Cytotoxic T Lymphocytes, which thus a) eradicate the transduced cells, resulting in transient expression of the transgene and b) cause inflammations (as described in numerous publications).

In addition, B cells are activated, leading to the production of antibodies against the adenoviral vectors (e.g. Yang et al. (1995), J. Virol. 69, 2004 - 2015).
As this adverse reaction is hampering gene therapy, several, solutions to this problem have been suggested, such as
a) using immune-suppressive agents after treatment and
b) disarming adenoviral vectors by e.g. additional deletion of E2A (Lusky *et al.*, J. Virol. 72: 2022, 1998 and references therein), E2B (e.g. Amalfitano et al. Proc. Natl. Acad. Sci. USA 93: 3352, 1996), E4 (Lusky *et al.*, J. Virol. 72: 2022, 1998 and references therein) or generation of so-called minimal vectors that do not contain any adenoviral gene (e.g. Kochanek et al. Proc. Natl. Acad. Sci. USA 93: 5731, 1996), and
c) equipping the adenoviral vectors with immuno-suppressing genes and
d) inhibition of co-stimulatory interactions between T cells and B cells (e.g. Kay et al. Proc. Natl. Acad. Sci. USA 94: 4686, 1997).

Ad. A: In one approach to suppress these reactions, attempts have been made to control the inflammatory response by adding glucocorticoids prior to and/or for a limited period of time after gene transfer. Pulmonary gene transfer was tested in the absence or presence of the potent anti-inflammatory agent dexamethasone. Dexamethasone significantly reduced adenovirus-associated inflammation after virus administration in animals and significantly increased gene transfer efficiency (β-galactosidase). All adenovirus-exposed animals had elevated levels of the chemo-attractive chemokines MIP-1alpha and MCP-1, as well as several pro-inflammatory cytokines including TNF-alpha, IL-6, IL-lbeta, and IFN-gamma. These levels were modulated by dexamethasone (Otake et al. Hum. Gene Ther. 10: 2207, 1998). Similar strategies using other components have been employed, including the use of the immuno-suppressants FK506, cyclophosphamide and cyclosporin.

Ad b: Further disarming of the adenoviral vectors, including the generation of minimal vectors, has been performed by many different groups. These attempts are all aiming at reducing or preventing the production of adenoviral proteins.

Ad c: Equipping the adenoviral vectors with immuno-suppressing genes. Among these immuno-suppressive genes are IkappaB-alpha super-suppresser (Ad.IkappaBM), aiming at suppressing NF-kappaB which is a key regulator of the innate anti-viral immune response. The vector was tested in bcl-2 transgenic mice. Combined action of IkappaBM and Bcl-2 allowed for vector persistence in livers, supporting the idea that the suppression of innate defence mechanisms improves vector persistence (Lieber et al. J. Virol. 72: 9267, 1998). For this purposes, also replication-deficient adenovirus vectors containing human catalase cDNA have been constructed. Catalase expression conferred on human and pig islets a reduction of non-specific inflammation effects on human or porcine islet grafts (Benhamou et al., Diabetologia 41: 1093, 1998).
Another example comprises the use of a vector containing a soluble TNF receptor (TNF-bp), which reduced the inflammatory response, and resulted in prolonged transgene expression in the liver and lung after either intravenous or intra-nasal administration of adenovirus. Treatment with TNF-bp reduced circulating levels of TNF-alpha, but had no effect on anti-adenovirus antibodies and induction of cytotoxic T cells 30 days after administration (Zhang et al., Hum. Gene Ther. 9: 1875, 1998).
In addition, natural adenoviruses contain an E3 region, which is involved in the evasion of the host immune response (for review see Wold et al. In: Viroceptors, Virokines and related Immune Modulators encoded by DNA viruses (1995), McFadden, editor, p147). Proteins encoded by the genes in this area have molecular weights of 19, 14.7, 11.6, 10.4 and 6.7 kDa.. None of the E3 proteins is required for adenovirus replication.
A function of the 19-kDa protein, called gp 19K because it is a glycoprotein, is to protect adenovirus-infected cells against MHC class-I restricted cytotoxic T-cell lysis. This glycoprotein complexes intra-cellularly with Class I histocompatibility antigens, thereby reducing recognition of the infected cell by the cellular immune system.
Another protein of the E3 region (14.7 kDa) is responsible for suppression of cytolysis induced by TNF. TNF is secreted by activated macrophages and lymphocytes and is cytotoxic or cytostatic to certain tumour cells. TNF also lyses cells infected with certain viruses and is released during infections by influenza virus. It has been shown that mouse C3HA fibroblasts are lysed by TNF when infected by adenovirus mutants that lack region E3. Uninfected cells are not lysed by TNF, nor are cells infected by wild-type adenovirus. Mutant recombinant adenoviruses that do not express E3-10.4K, E3-14.5K and E3-14.7K induced increased infiltration of neutrophils.
The 10.4 kDa and 14.5 kDa proteins encoded by the E3 region function in concert to down-regulate the EGF receptor (tyrosine-kinase) in adenovirus-infected cells. Stimulation of the kinase activity of EGF-R results in the activation of cellular metabolism and eventually in the induction of DNA synthesis and mitosis. The biological significance of EGF-R down-regulation is unknown.
To limit the immune responses, adenoviral vectors have been constructed in which the whole E3 region or part of it, is retained. In some of these vectors, the E3 genes were expressed to very high levels by using heterologous promoters. Other viruses, like Herpes Simplex Virus, also contain genes involved in modulating the host immune response to viral infection. An example is the Herpes Simplex Virus gene ICP47, which like gpl9K of adenovirus, down-regulates MHCI peptide expression on the cell surface. Adenoviral vectors have been constructed harbouring the ICP47 gene, which turned out to be efficient in preventing MHC-I expression on the cell surface of the infected cells (York et al., Cell 77: 525, 1994).

Ad d: inhibition of co-stimulatory interactions between T cells and B cells.
Cellular immunity dependent on CD4+ and CD8+ T cells contributes to the loss of recombinant gene expression and the development of localised inflammation. Antigen specific activation of T cells occurs to both viral proteins and the transgene product. Systemic levels of neutralising antibody to the capsid proteins of the vector are also generated. Strategies are developed to prevent the cellular and humoral immunity to this therapy based on transiently ablating CD4+ T cell activation at the time of vector delivery.
In an approach to overcome this CTL-mediated immune response elicited by gene therapy, interactions between T cells and B cells/antigen-presenting cells were transiently blocked around the time of vector administration. Treatment at the time of primary-vector administration with a monoclonal antibody against murine CD40 ligand, combined with administration of an adenovirus vector carrying murine CTLA4Ig, an inhibitor of the CD28/B7 pathway, prolonged transgene (β-galactosidase) expression in airways for up to 28 days and resulted in persistent alveolar expression for >90 days. This treatment regimen reduced local inflammation and markedly reduced the T-cell and T-cell-dependent antibody response to the vector (Wilson et al., J. Virol. 72: 7542, 1998). In has also been proposed to inhibit CD4+ T cell activation by transiently administering CTLA4Ig at the time an E1-deleted adenovirus to liver or lung. In lung, CTLA4Ig. treatment significantly blocked the formation of neutralising antibodies, allowing efficient re-administration of virus, whereas transgene expression was only moderately prolonged. In vitro assays revealed suppression of T cell, suggesting that transient inhibition of the CD28/B7 pathway at the time of virus instillation can partially interfere with the immune response to adenovirus-mediated gene transfer (Jooss et al. Gene Ther. 5: 309, 1998). Encouraging results were obtained when vector was administered with one of several immune modulating agents including a MAb to CD4+ cells, and MAb to CD40 ligand. Transient ablation of CD4+ T cell activation prevents the responses of the CD8+ T and B cells (Yang et al Hum. Mol. Genet. 5: 1703, 1996).

### SUMMARY OF THE INVENTION

The invention provides means and methods for at least in part preventing an immune response to certain cells in a body while leaving the general capacity of the immune system to respond to other antigens and cells essentially intact.

### BRIEF DESCRIPTION OF THE DRAWINGS

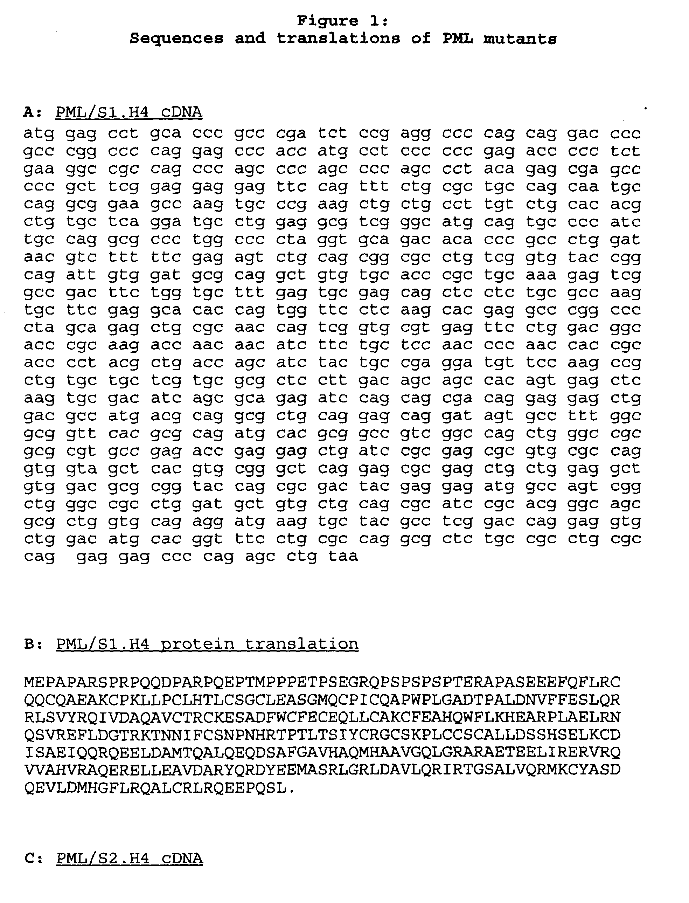

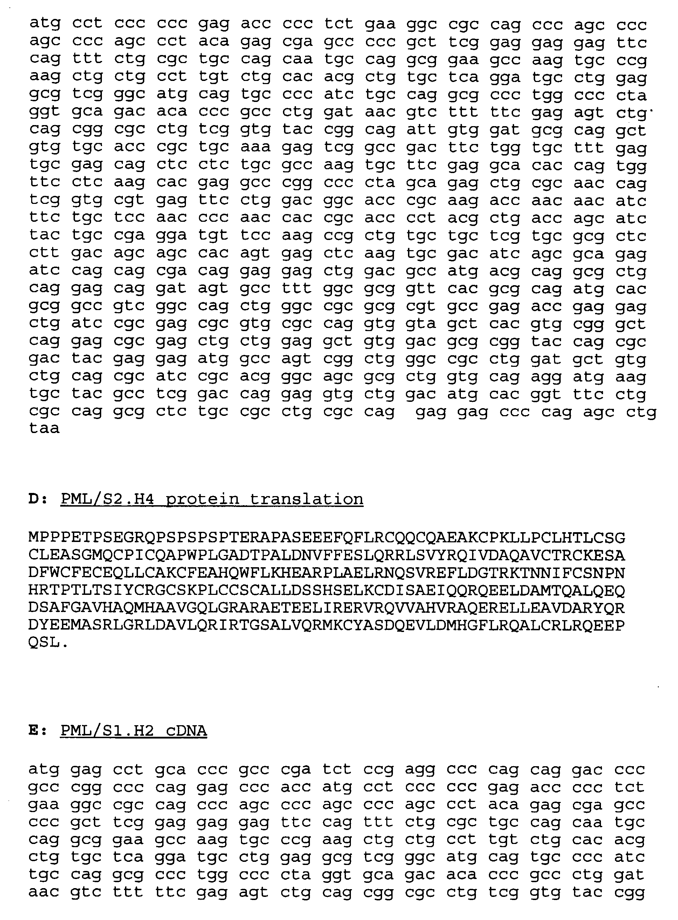

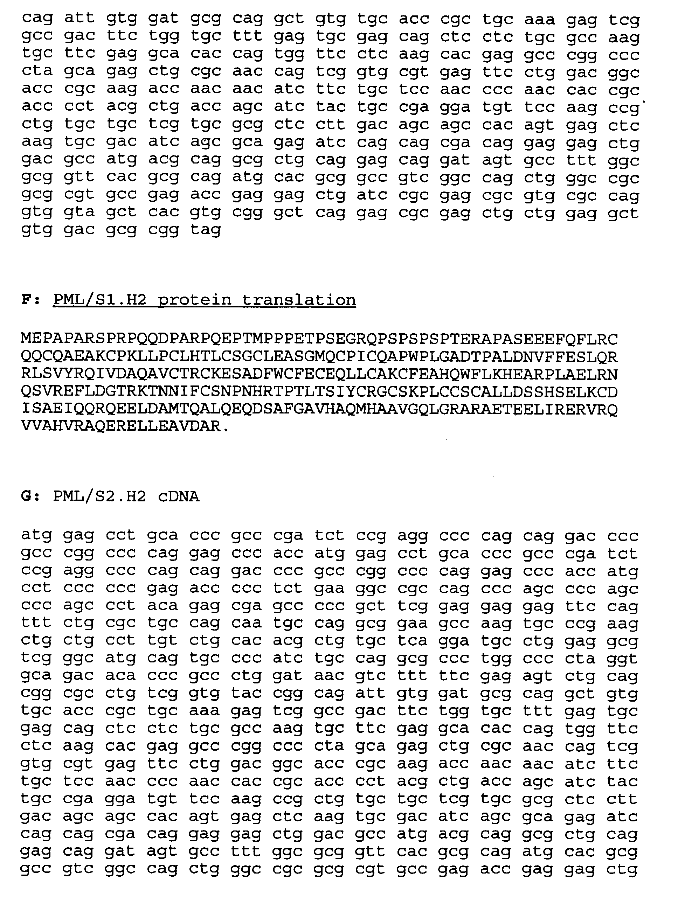

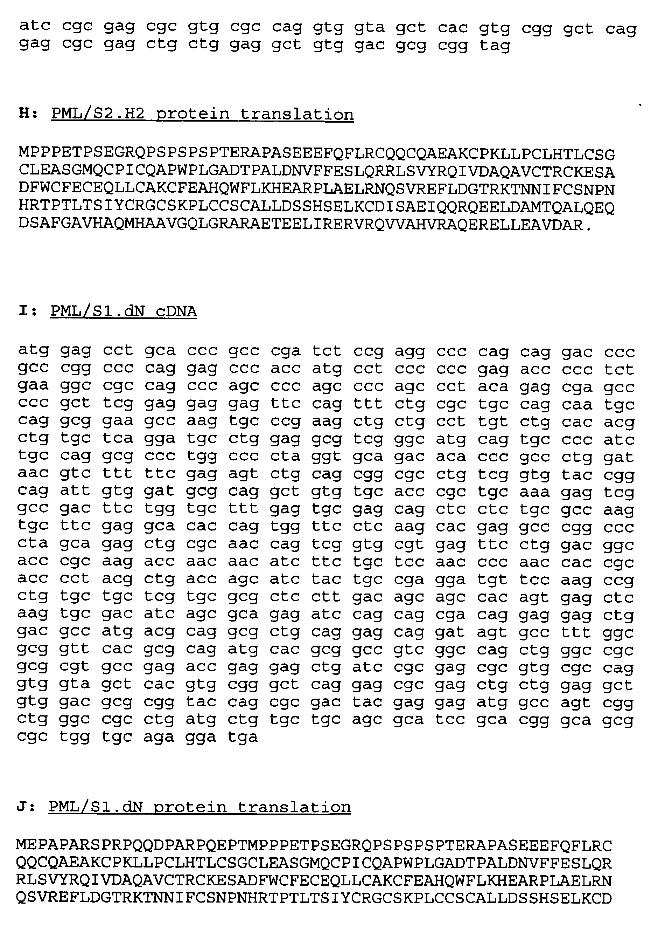

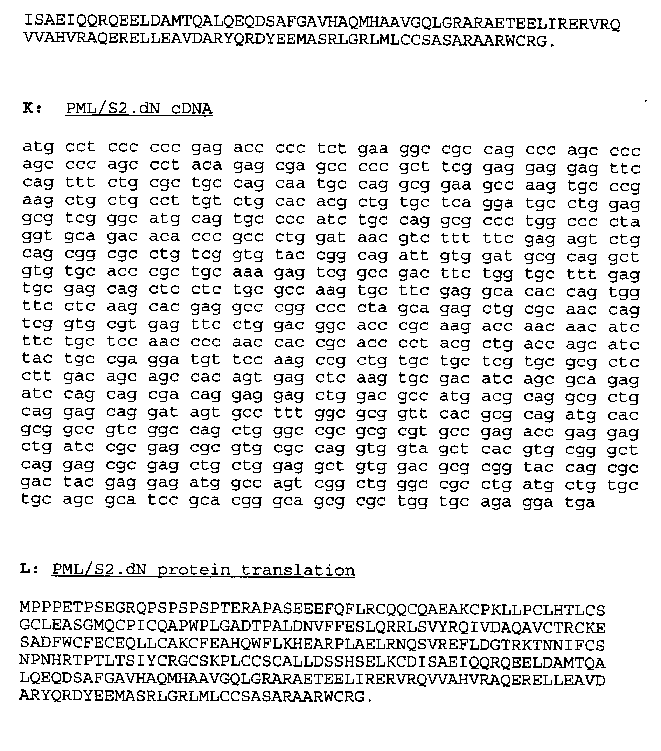

Figure 1. Nucleotide and amino-acid sequence of some human PML mutants.

### DETAILED DESCRIPTION OF THE INVENTION

The natural immune mediated removal of cells from the body of an individual is sometimes undesired. For instance in gene therapy or transplantation medicine where removal of gene modified or transplanted cells or both counteracts the therapy. Another example is auto-immune disease, in auto-immune disease the immune system is incorrectly removing normal healthy cells from the body leading to auto-immune disease related symptoms. Methods to suppress the general activity of the immune system in an individual are undesired since these methods also affect favourable aspects of the immune system such as the capacity of the immune system to respond to common virus and/or bacterial infections.

In one aspect the present invention provides a method for preventing at least in part the action of the immune system toward a specific set of cells while leaving the capacity of the immune system to react to other cells or antigens intact comprising providing said cells with a gene delivery vehicle having been provided with a nucleic acid encoding at least one protein capable of at least in part inhibiting peptide presentation by major histocompatibility complex I.

With the means and methods of the invention a person skilled in the art will be able to modify specific cells and prevent at least in part an immune related removal of said cells in a mammalian body. The invention is useful for preventing immune related removal of for instance transplanted cells, gene modified cells and cells inadvertently targeted for removal in auto-immune related diseases. In one aspect the invention provides gene therapy vehicles that can at least in part confer to cells the capacity to evade the cellular immune system. The invention further provides gene therapy vehicles comprising a nucleic acid with therapeutic properties that can further confer to cells transduced with said nucleic acid the capacity to at least in part evade the cellular immune system.

Gene delivery vehicles of the current invention do not. essentially systemically affect the general potency of the immune system, as is the case when immuno-suppressive agents are used or if secreted immuno-suppressive agents like TNF-alpha receptor are used.
Preferably but not necessarily said gene delivery vehicles comprise nucleic acid that does not encode additional viral proteins or does not allow expression of viral proteins (which are non-self and, therefore, can be immunogenic). Preferably but not necessarily peptide presentation by major histocompatibility complex I is at least in part inhibited by inhibiting transcription of genes involved in MHC peptide presentation. It would clearly be advantageous to have gene therapy vectors available that do not elicit an immune response, and yet retain all transducing capabilities of the current vectors. It is an object of the present invention to provide novel vectors that improve persistence and diminish pathology.
The invention also provides methods and materials to locally, and around the time of vector administration suppress the immune response, in order to overcome the limitations of current gene therapy vectors.
In one embodiment of the invention said inhibition of peptide presentation by major histocompatibility complex I by cells is provided by supplying cells with a nucleic acid encoding a mutant PML.

In spontaneous tumours and many virus transformed cell lines, the genes involved in MHC class I presentation, i.e. encoding class I heavy chain, TAP-1 and TAP-2, LMP-2 and LMP-7 are simultaneously repressed. Recently, the proto-oncogene PML has been identified as a critical regulator for the expression of the genes involved in MHC class I presentation (40).

The PML gene, also designated *myl* , was first described by cloning of the translocation break points of a reciprocal t(15;17) translocation in acute promyelocytic leukemia (APL) (26-28). This translocation resulted in a fusion between most of the N-terminal part of the PML gene on chromosome 15 with all but the N-terminal part of the RAR-a gene on chromosome 17 (29-31). The structure of the PML encoded protein is highly conserved between mouse and man (29-32). The N-terminal region of PML, encoded by exon 1, 2 and 3 of the PML gene (33), harbours three cysteine rich metal binding domains, forming a RING finger domain preceding two 'B-box' fingers (34). These structures have putative functions involving some aspects of transcription regulation (35-37). The metal binding domains are followed by a predicted coiled-coil domain comprising 4 α-helices, which is thought to be involved in dimer formation of PML proteins (37-39). The PML protein is localised in PML Oncogenic Domains (PODs), nuclear bodies that contain multiple proteins (reviewed in 39). Both the cysteine rich region and the α-helical region are thought to be required for the localisation in PODs (34,37 and 39), supporting a model that PML can interact with multiple proteins, both via α-helical structure and the RING finger domain. Interestingly, all alternatively spliced PML mRNAs described thus far contain the sequences from exon 1, 2 and 3, which encode the above-described functional domains (33). This suggests that these domains play a crucial role the function of PML. Mutation or deletion of parts of these functional domains could therefore yield interesting truncated PML proteins with different activities, e.g. dominant negative mutants.

PML has recently been identified as a critical regulator for the expression of the genes involved in MHC class I presentation (40). Analysis of recurrent tumours that arose in mice despite a strong CTL response against the unmutated tumour antigen P1A, revealed a unique deletion in exon-3 of PML (G at position 962), F12dG. This deletion caused a frame shift, which results in pre-mature termination of translational of the PML protein. The truncated protein consists of the first 292 amino acids of PML, harbouring the RING finger and a part of the α-helical coiled coil structure, followed by a short peptide with a new sequence (KLCCSASALVGRWSRG). Transfection of this naturally occurring mutant of PML into the parental tumour cells (J558-B7) resulted in down-regulation of both the TAP genes and the LMP genes, resulting in strongly reduced MHC class I expression in the transfectants (40). The mode of action of this dominant negative mutant of PML is still unclear, but, since the major part of the coiled coil is still present in this mutant, this protein may dimerise with wild type PML, thereby interfering with its function.
Gene therapy vectors expressing PML mutants that are able to down-modulate the expression of MHC class I molecules on surface of the transduced cells can be used in transplantation medicine. As a non-limiting example, pancreatic islet β-cells from an MHC class I mismatched donor can be transduced with a gene therapy vector expressing dominant negative PML before being transplanted into a recipient. This results in at least a partial down-regulation of the cell surface expression of MHC class I molecules of the donor cells, thereby at least partially circumventing the MHC class I haplotype matching requirements between donor and recipient. In spite of the genetic mismatch of the MHC class I haplotype, the graft will then at least partially not be rejected by the cellular immune system of the recipient. This example can be extended to kidney-, skin-, heart-, liver- and lung transplantation, etc., etc.. During the adenovirus lytic infection cycle, the POD structures in the nucleus of the infected cells are re-organised (Puvion-Dutilleul et al., Exp. Cell. Res. 218: 9, 1995) Adenovirus infection causes drastic redistribution of. PML from spherical nuclear bodies, i.e. PODs, into fibrous structures (Carvalho et al., J. Cell Biol. 131: 45, 1995), also referred to as tracks' (Doucas et al., Genes Dev. 10 : 196, 1996). Re-organisation of the PODs turned out to be a function of the early region 4 from the adenovirus. Early region 4 encodes several polypeptides that are translated from a differentially spliced E4-transcribed RNA. One or more functions from these polypeptides are pivotal for progression through the lytic infection cycle, but the exact function of all the individual E4-encoded polypeptides has not been defined yet. Interestingly, two of the E4 products, i.e. the polypeptides encoded by orf-3 and orf-6, can partially or totally compensate for deficiencies in each other (Bridge et al., Virol. 174: 345, 1989; Huang et al., J. Virol. 63: 2605, 1989).

Both the Orf-3 and Orf-6 encoded proteins are involved in processes that increase viral late protein production by facilitating the cytoplasmic accumulation of the relevant mRNAs encoding the adenoviral late proteins. They exert their function at the post-transcriptional level (reviewed in Leppard, J. Gen. Virol., 78, 2131, 1997). In complex with the 55K-E1B protein, E4-orf6 does so by increasing the export rate of the relevant mRNAs from the nucleus. The E4-orf6/E1B-55K complex is located in so called viral inclusion bodies, the region where viral DNA replication, viral late gene transcription and RNA processing occur (Pombo et al., EMBO J., 13: 5075, 1994). In addition to that, both E4-orf6 and E4-orf3 improve the stability of the unprocessed late RNA, thereby increasing the pool of RNA that is available for post-transcriptional processing and increasing splicing and RNA transport processes. Finally, both the E4-orf6 and E4-orf3 encoded proteins are required for adenoviral DNA synthesis.

Recently, it has been suggested that E4-encoded proteins directly affect the distribution of a group of essential transcription/replication factors that are normally present in POD structures in the nucleus of infected cells (reviewed in Leppard, J. Gen. Virol., 78, 2131, 1997). Analysis of Adenovirus mutants revealed that double mutation of E4-orf3 and E4-orf6 prevents reorganisation of the PODs. Mutation of E4-orf6 turned out only to delay the reorganisation of PODs and the formation of tracks. However, mutation of E4-orf3 both delayed and severely decreased the track formation (Doucas et al., Genes Dev. 10 : 196, 1996). Interestingly, E4-orf3 co-localised with the PML protein, suggesting that interaction between those proteins is pivotal for POD disruption and track formation. This hypothesis is strengthened by the observation that overexpression of wild type PML inhibited E4-orf3 induced POD disruption and track-formation. Moreover, treatment of cells with interferon, a strong inducer of PML expression, inhibits POD reorganisation and inhibits DNA replication in the viral inclusion bodies. Co-localisation of E4-orf3 and PML required the α-helical domain of PML. PML mutants that are deleted for the α-helical domain did not co-localise with E4-orf3, showing that this domain of PML plays a crucial role in this process (Doucas et al., Genes Dev. 10 : 196, 1996). Although much of the importance of POD disruption and track formation remains to be established, it is clear that this leads to redistribution of factors, such as Sp-100, NDP-55 and possibly other POD associated proteins into the viral inclusion bodies. Most likely, a number or all of the POD associated proteins are required for viral DNA replication and or viral RNA processing and have to be recruited to the viral inclusion bodies for progression through the adenoviral life cycle.

Given the fact :
1) that POD disruption and track formation is an event in the adenovirus life cycle and
2) that the α-helical domain of PML is at least in part required both for E4-orf3 association and the subsequent POD re-organisation,
a dominant negative mutant of PML as described in this invention, e.g. a mutant that contains the essential domain of PML for dimerisation but is at least part deficient for other functions of said PML protein. Such a dominant negative mutant or a functional part, derivative or analogue thereof, might exert similar function as E4 encoded proteins such as E4-orf3. Therefore, a cell line overexpressing the dominant negative form of PML might be used to produce adenoviral vectors that are partially or completely deleted for early region 4.

In one aspect the invention provides a method for at least in part inhibiting an immune response against specific cells in a body without essentially affecting the capacity of the immune system in said body to mount other immune responses, comprising providing said cells with a gene delivery vehicle comprising a nucleic acid sequence encoding at least one protein capable of at least in part inhibiting peptide presentation by major histocompatibility complex I. Preferably said protein exhibits two or more activities inhibiting peptide presentation by major histocompatibility complex I. In one embodiment at least part of said protein is derived from a protein naturally present in a cell, preferably a eukaryotic cell. In another embodiment at least part of said protein is derived from a protein naturally present in a mammalian cell. In yet another embodiment at least part of said protein is derived from a protein naturally present in a human cell. In another embodiment said protein is a mutant PML or functional part, derivative or analogue thereof. Preferably said protein is derived from human PML or a functional part, derivative or analogue thereof. In another embodiment said mutant PML comprises an amino-acid sequence of PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L) or functional part, derivative or analogue thereof. Preferably said mutant PML is derived from a mammal, preferably from a primate or a rodent. Particularly preferred is a mutant PML derived from a human.
In one aspect said mutant PML comprises N-terminal sequences of PML up until the first α-helix of the coiled-coil structure or a functional part, derivative or analogue thereof. In another aspect said mutant PML comprises N-terminal sequences of PML or a functional part, derivative or analogue thereof and at least one of the α-helices of the coiled-coil structure or a functional part, derivative or analogue thereof. In another aspect said mutant PML comprises N-terminal sequences of PML or a functional part, derivative or analogue thereof and at least two of the α-helices of the coiled-coil structure or a functional part, derivative or analogue thereof. In another aspect said mutant PML comprises N-terminal sequences of PML or a functional part, derivative or analogue thereof and at least three of the α-helices of the coiled-coil structure or a functional part, derivative or analogue thereof. In another aspect said mutant PML comprises N-terminal sequences of PML or a functional part, derivative or analogue thereof and at least four of the α-helices of the coiled-coil structure or a functional part, derivative or analogue thereof. In another aspect said mutant PML comprises at least one of the α-helices of the coiled-coil structure or a functional part, derivative or analogue thereof. In one embodiment of the invention said cells comprise cells of a transplant, where said method improves at least in part the survival of said cells in said individual, wherein said individual is a recipient of said transplant.
In another embodiment of the invention said cells comprise target cells of an auto-immune response, where said method improves at least in part the survival of said cells in said individual. In one non-limiting embodiment said cells of transplant or said target cells of an auto-immune response comprise pancreatic islet β-cells or Langerhans cells. It can be envisaged that cells correctly targeted for removal from the body by the immune system, for instance due to the fact that they are expressing a foreign virus antigen, are at least in part protected by a method of the invention. Such protection may become desirable for instance when cells expressing said foreign antigen are performing a vital function. One non-limiting example is when an entire organ or a decisive part thereof of a patient for instance the liver is infected with a virus and wherein said infection is not detrimental for liver cells or life threatening for a patient, but where it is the removal from the body of the virus infected cells triggered by the immune system that is life threatening. In situations like this, the immune system is in principle correctly targeted toward the virus infected cells since a limited infection would be best abated by removal of the infected cells from the body. However, in cases where the infection has proceeded to far and to many cells have become infected, in some cases the best treatment would be to avoid or at least in inhibit the immune response against at least some of the virus infected cells. Another non-limiting example where cells expressing a foreign antigen, are best at least in part protected by a method of the invention is found in at least some gene therapy applications. In most gene therapy applications cells provided with a nucleic acid express an antigen that is foreign to the individual receiving gene therapy. In some instances, for instance when prolonged expression of a transgene in certain cells of an individual is desired, one would like to be able to at least in part diminish the capacity of the immune system of said individual to respond to said cells.
In one embodiment of the invention said cells of a transplant or target cells of an auto-immune response or other cells are further provided with a nucleic acid encoding at least one protein with a different activity, preferably a therapeutic activity. Preferably said protein with a therapeutic activity is a nitric oxide synthetase or a part, derivative or functional analogue thereof. In another preferred embodiment said protein with a therapeutic activity is a secreted protein such as but not limited to interleukin-3 or a part, derivative or functional analogue thereof.
In yet another embodiment said cells are further provided with a nucleic acid encoding at least one additional protein capable of providing cells with the capacity to at least in part evade an immune response. Preferably but not necessarily said capacity to at least in part evade an immune response is due to an activity that at least in part inhibits peptide presentation by major histocompatibility complex I. Inhibition of peptide presentation by major histocompatibility complex I (MHC-I) may be achieved through any means. Including preventing peptides from interacting with MHC-I or preventing transport of MHC-I to the cell surface or other means such as preventing MHC-I with or without a peptide from interacting with other molecules. In yet another embodiment of the invention the capacity of the immune system in said body to mount an immune response against said cells in said body, is additionally at least in part inhibited through administering to said body at least one compound capable of inhibiting immune responses in an essentially non-specific way. The means and methods of the invention can be used to at least in part decrease the amount of compound capable of inhibiting immune responses in an essentially non-specific way, such as but not limited to cyclosporin A, needed to prevent at least in part an immune response against specific cells in a body.

In another aspect the invention provides a nucleic acid delivery vehicle having been provided with a nucleic acid sequence encoding at least one protein capable of at least in part inhibiting peptide presentation by major histocompatibility complex I. In a preferred embodiment said protein exhibits two or more activities inhibiting peptide presentation by major histocompatibility complex I. In a preferred embodiment said protein is at least in part derived from a eukaryotic cell, preferably a mammalian cell, preferably a human or rodent cell or functional analogue thereof. In a preferred aspect said protein comprises a mutant PML or a functional part, derivative or analogue thereof. Preferably said mutant PML comprises an amino-acid sequence of PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L) or functional part, derivative or analogue thereof. In a preferred aspect expression of a protein capable of at least in part inhibiting peptide presentation by major histocompatibility complex I, preferably in two or more ways, is under control of a promoter of which the activity can be regulated. Activity may be regulated through the use of a promoter of which the activity can be regulated with a signal such as but not limited to promoters of which the activity can be induced or repressed following the addition of a compound. Activity may also be regulated through the use of a promoter that has an inherent variable activity for instance but not limited to a promoter susceptible to inactivation for instance through methylation. A promoter of which the activity can be regulated is useful for, for instance, the induction of tolerance for the cells treated with means and or methods of the invention (i.e. when the immune system of the host no longer sees the antigens presented by said cells as foreign). When the host has become tolerant for cells that were treated with means and/or methods of the invention, it is preferred that said cells are at least in part deinhibited from peptide presentation by major histocompatibility complex I, so as to allow the immune system at least in part to clear also at least part of said cells when said cells express a novel foreign antigen, such as would be the case in a normal virus infection.
In one embodiment the invention provides a nucleic acid delivery vehicle of the invention wherein said vehicle further comprises a nucleic acid encoding at least one protein with a different activity, preferably a therapeutic activity. Preferably said protein with a different activity is a nitric oxide synthetase or a part, derivative or functional analogue thereof. In another preferred embodiment said protein with a different activity is a secreted protein such as interleukin-3 or a part, derivative or functional analogue thereof. In another preferred embodiment is provided a nucleic acid delivery vehicle of the invention further provided with a nucleic acid sequence encoding a herpes simplex virus thymidine kinase or a part, derivative or functional analogue thereof. In this preferred embodiment expression of said protein enables, at least in part, killing of cells provided with the means and/or methods of the invention through exposure of said cells to effective amounts of gancyclovir and provides a safety measure against inadvertent undesirable side effects caused by the effect of means and/or methods of the invention.
In one embodiment the invention provides a nucleic acid delivery vehicle according to the invention, further comprising a nucleic acid sequence encoding at least one additional protein capable of providing cells with the capacity to at least in part evade an immune response. In one aspect is provided a nucleic acid delivery vehicle of the invention, wherein said vehicle has at least in part been provided with a tissue tropism for smooth muscle cells and/or endothelial cells. In another aspect is provided a nucleic acid delivery vehicle of the invention, wherein said vehicle has at least in part been deprived of tissue tropism for liver cells. In a preferred embodiment said changed tissue tropism is provided by at least a tissue tropism determining part of a fiber protein of a group B adenovirus, preferably adenovirus 16.
In one aspect a nucleic acid delivery vehicle of the invention comprises a nucleic acid comprising integration means of an adeno-associated virus nucleic acid. Said integration means comprise at least one adeno-associated virus inverted terminal repeat sequence. Preferably said integration means are comprise at least two adeno-associated virus inverted terminal repeat sequences wherein preferable one adeno-associated virus inverted terminal repeat sequence is present on either side of a nucleic acid sequence of which integration into the host chromosome is desired.
Said nucleic acid delivery vehicle may be any kind of nucleic acid delivery vehicle including non-viral or viral or a combination of both types of nucleic acid delivery vehicles. Said nucleic acid delivery vehicle may even comprise water, in cases where nucleic acid is injected into a body through one or more needles. In a preferred embodiment a nucleic acid delivery vehicle according to the invention is an adenovirus particle, an adeno-associated virus particle or a retrovirus particle. Said adenovirus particle may be derived from any type of adenovirus. Preferably said adenovirus particle is derived from adenovirus 5 and/or adenovirus 16. Said adeno-associated virus particle or integration means may be derived from any type of adeno-associated virus. Preferably said adeno-associated virus particle or integration means are derived from adeno-associated virus 2. Said retrovirus particle may derived from any type of retrovirus. Preferably said retrovirus particle is derived from a murine leukemia virus or a lentivirus, preferably human immunodeficiency virus.
In a preferred embodiment said adenovirus particle comprises an adenovirus vector. Said adenovirus vector may be derived from any type of adenovirus. Preferably said adenovirus vector is derived from adenovirus 5. Said adenovirus vector may at least in part deprived of the capability to express E1-region encoded proteins, preferably through a deletion in the E1-region. Said vector may at least in part be deprived of the capability to express E2A protein, preferably through at least a partial deletion of nucleic acid encoding said E2A protein. Said vector may it least in part be deprived of the capability to express at least one of the E3-region encoded proteins, preferably through at least a partial deletion of nucleic acid encoding at least one of said E3-region encoded proteins. Said vector may at least in part be deprived of the capability to express at least one of the E4-region encoded proteins, preferably E4-Orf3 and/or E4-Orf6, preferably through at least a partial deletion of nucleic acid encoding at least one of said E4-region encoded proteins.
Said vector may also be deprived of the capacity to express a combination of one or more of the proteins encoded by said regions or by said E2A-gene.
In another aspect the invention provides a cell for the production of an adenovirus particle of the invention comprising one or more means for the complementation of at least part of the function of one or more of the proteins said vector has at least in part been deprived the capability of expression of. Preferably said cell is derived from a PER.C6 cell (ECACC deposit number 96022940).
In another aspect the invention provides means to at least in part complement the function of at least part of the proteins encoded by the E4-region in an adenovirus vector. For instance but not limited to, complementing replication functions of an adenovirus vector during replication of an adenovirus vector, for instance during production of an adenovirus vector in a cell. Said means including the expression of a mutant PML or functional part, derivative or analogue thereof of the invention, for instance in a cell producing an adenovirus vector deprived at least in part of the capacity to express one or more proteins encoded by the E4-region. Preferably E4-region encoded protein at least in part complemented by mutant PML or functional analogue thereof is E4-orf3, E4-orf6 or both. Complementation of an adenovirus vector at least in part deprived of the capacity to express at least part of the proteins encoded by the E4-region may be achieved by a mutant PML or functional analogue thereof encoded by nucleic acid present in the chromosomal DNA. However, said mutant PML or functional analogue thereof may also be encoded by nucleic acid present on nucleic acid of said adenovirus virus. Nucleic acid encoding a mutant PML or functional analogue thereof may also be provided by other means or alternatively mutant PML or functional analogue thereof may be provided in other ways. Expression of a mutant PML or functional analogue thereof in a cell producing adenovirus particles comprising an adenovirus vector, deprived at least in part of the capacity to express E4-region encoded proteins or not, may lead to increased production of said adenovirus particles.
In yet another aspect the invention provides a cell comprising a mutant PML or functional part, derivative or analogue of the invention.
In yet another aspect the invention provides a use of a protein capable of inhibiting peptide presentation by major histocompatibility complex I, for at least in part preventing immune response related removal of a selected population of cells from a body without essentially affecting the capacity of the immune system in said body to mount other immune responses. Preferably said protein exhibits two or more activities inhibiting said peptide presentation. Preferably said protein is a mutant PML or functional part, derivative or analogue of the invention, preferably comprising an amino-acid sequence of PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L) or functional part, derivative or analogue thereof. In another aspect the invention provides a use of a mutant PML or functional part, derivative or analogue thereof, preferably comprising an amino-acid sequence of PML-mutant F12dG or functional part, derivative or analogue thereof, in a medicament.
In one embodiment the invention provides a functional analogue to PML-mutant F12dG, derived from a primate. In another embodiment the invention provides a nucleotide sequence encoding a primate derived functional analogue of such as PML-mutant F12dG, PML.S1.H4 (figure 1A), PML/S2.H4 (figure 1C), PML/S1.H2 (figure 1E), PML/S2.H2 (figure 1G), PML/S1.dN (figure 1I) or PML/S2.dN (figure 1K) or a functional part, derivative or analogue thereof.
In one embodiment the invention provides an adenovirus vector comprising a nucleic acid sequence encoding a protein capable of at least in part inhibiting peptide presentation by major histocompatibility complex 1.
In another embodiment the invention provides an adeno-associated virus vector comprising a nucleic acid sequence encoding a protein capable of at least in part inhibiting peptide presentation by major histocompatibility complex 1. In another embodiment the invention provides a retroviral vector comprising a nucleic acid sequence encoding a protein capable of at least in part inhibiting peptide presentation by major histocompatibility complex 1.
In another embodiment the invention provides an adenovirus vector, and adeno-associated virus vector or a retrovirus vector comprising a nucleic acid sequence encoding a mutant PML or functional part, derivative or analogue thereof. In another embodiment the invention provides a primate cell comprising a mutant PML or a functional part, derivative or analogue thereof.
Activation of cytotoxic T lymphocytes (CTLs) to cells infected with adenovirus vectors contributes to problems of inflammation and transient gene expression that attend their use in gene therapy. One aspect of the present invention discloses the use of a PML-mutant F12dG or functional homologues thereof, preferably a dominant negative regulator of key genes involved in MHC class I mediated antigen presentation, for the design of new recombinant gene therapy vectors. Following transduction of target cells, expression of a PML-mutant F12dG or functional homologues thereof at least in part prevents presentation of peptides derived from components of the vector itself or the transgene product to the immune system, thereby at least in part preventing an immune response against the transduced cells.
In a further embodiment of the present invention, adenoviral vectors are provided that lack both El and E2A sequences and harbour a PML-mutant F12dG or functional homologues thereof. In addition, adenoviral vectors are provided that lack both E1 and E4 or E1, E2A and E4, or vectors that are deleted for other early or late functions, or vectors that are deleted for all adenoviral genes. Moreover, adenoviral vectors are provided that lack both E1 and E4 or E1, E2A and E4 and, in addition, have reduced expression of the other early genes and/or late genes.

### EXAMPLES

### Example 1

Plasmid based system for the generation of PML expressing recombinant adenoviral vectors deleted in early region 1 and/or early region 2A expressing
A dominant mutant of PML
Generation of the adapter plasmid pAd5/CLIP.PMLdN: Adapter plasmid pMLP.TK (patent application EP 95202213) was modified as follows: SV40 polyA sequences were amplified with primer SV40-1 (introduces a BamHI site) and SV40-2 (introduces a BglII site). In addition, Ad5 sequences present in this construct were amplified with primers Ad5-1 (introduces a BglII site) and Ad5-2. 
SV40-1: 5'-GGGGGATCCGAACTTGTTTATTGCAGC-3'
SV40-2: 5'-GGGAGATCTAGACATGATAAGATAC-3'
Ad5-1: 5'-GGGAGATCTGTACTGAAATGTGTGGGC-3'
Ad5-2: 5'-GGAGGCTGCAGTCTCCAACGGCGT-3'
 Both pcr fragments were digested with BglII and ligated. The ligation product was amplified with primers SV40-1 and Ad5-2 and digested with BamHI and AflII. The digested fragment was then ligated into pMLP.TK pre-digested with the same enzymes. The resulting construct, named pMLPI.TK, contains a deletion in adenovirus E1 sequences from nt. 459 to nt. 3510.

This plasmid was used as the starting material to make a new vector in which nucleic acid molecules comprising specific promoter and gene sequences can be easily exchanged. First, a PCR fragment was generated from pZipΔMo+PyF101(N⁻) template DNA (described in PCT/NL96/00195) with the following primers: 
LTR-1: 5'-CTG TAC GTA CCA GTG CAC TGG CCT AGG CAT GGA AAA ATA
CAT AAC TG-3'
LTR-2: 5'-GCG GAT CCT TCG AAC CAT GGT AAG CTT GGT ACC GCT AGC
GTT AAC CGG GCG ACT CAG TCA ATC G-3'.
 Pwo DNA polymerase (Boehringer Mannheim) was used according to manufacturers protocol with the following temperature cycles: once 5' at 95°C; 3' at 55°C; and 1' at 72°C, and 30 cycles of 1' at 95°C, 1' at 60°C, 1' at 72°C, followed by once 10' at 72°C. The PCR product was then digested with BamHI and ligated into pMLP10 (Levrero *et al.,* 1991; *Gene* 101, 195-202) digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Sequencing confirmed correct amplification of the LTR fragment. However, the most 5' bases in the pcr fragment were missing so that the PvuII site was not restored. Next, the coding region of the murine HSA gene was inserted. pLTR10 was digested with BstBI followed by Klenow treatment and digestion with NcoI. The HSA gene was obtained by PCR amplification on pUC18-HSA (Kay *et al.*, 1990; *J. Immunol.* 145, 1952-1959) using the following primers: 
HSA1, 5'-GCG CCA CCA TGG GCA GAG CGA TGG TGG C-3'
HSA2, 5'-GTT AGA TCT AAG CTT GTC GAC ATC GAT CTA CTA ACA GTA
GAG ATG TAG AA-3'.
 The 269 bp-amplified fragment was sub-cloned in a shuttle vector using the NcoI and BglII sites. Sequencing confirmed incorporation of the correct coding sequence of the HSA gene, but with an extra TAG insertion directly following the TAG stop codon. The coding region of the HSA gene, including the TAG duplication was then excised as a NcoI (sticky)-SalI (blunt) fragment and cloned into the 3.5 kb NcoI (sticky)/BstBI (blunt) fragment from pLTR10, resulting in pLTR-HSA10. Finally, pLTR-HSA10 was digested with EcoRI and BamHI, after which the fragment containing the left ITR, the packaging signal, the L420 promoter and the HSA gene was inserted into vector pMLPI.TK digested with the same enzymes and thereby replacing the promoter and gene sequences. This resulted in the new adapter plasmid pAd5/L420-HSA that contains convenient recognition sites for various restriction enzymes around the promoter and gene sequences. SnaBI and AvrII can be combined with HpaI, NheI, KpnI, HindIII to exchange promoter sequences, while the latter sites can be combined with the ClaI or BamHI sites 3' from HSA coding region to replace genes in this construct.

Another adapter plasmid that was designed to allow easy exchange of nucleic acid molecules was made by replacing the promoter, gene and polyA sequences in pAd5/L420-HSA with the CMV promoter, a multiple cloning site, an intron and a polyA signal. For this purpose, pAd/L420-HSA was digested with AvrII and BglII followed by treatment with Klenow to obtain blunt ends. The 5.1 kb fragment with pBr322 vector and adenoviral sequences was isolated and ligated to a blunt 1570 bp fragment from pcDNA1/amp (Invitrogen), obtained by digestion with HhaI and AvrII followed by treatment with T4 DNA polymerase. This adapter plasmid was named pAd5/Clip.

The coding sequence of PML-F12dG, which encodes a dominant negative mutant of mouse PML, harbours a deletion of G at position 962 as compared to the wild type PML coding sequences (Zheng et al., 1998, Nature 396: 373-376; Goddard et al., 1995, Mamm Genome 6, 732-737). This G(962)-residue is situated in exon 3 of the PML gene and its deletion causes a frame shift and premature translation termination of the PML protein (Zheng et al., 1998, Nature 396, 373-376 1998). The coding sequences of PML-F12dG can be amplified from the vector PML-F12dG (Zheng et al., 1998, Nature 396, 373-376) using the Expand 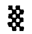 Long Template PCR system according to the standard protocol of the supplier (Boehringer Mannheim). The following primers can be used: 
PMLdN5: 5'- CCC AAG CTT GCC ACC ATG CCT CCC  CCA GAG GAA CCC.
PMLdN3: 5'- CGC GGA TCC GGT CGG AGG CAT AGA GCT TCA TCC TCT.
 PMLdN5 contains a HindIII restriction site (bold) for easy cloning and a Kozak sequence (underlined) for optimal translation of the messenger. PMLdN3 contains a BamHI restriction site (bold) for easy cloning. The amplified DNA fragment of approximately 950 bp will subsequently be digested with HindIII and BamHI. Next, the digested PCR fragment will be ligated into the HindIII/BamHI digested pAd5/CLIP vector, yielding pAd5/CLIP.PMLdN. The integrity of the PMLdN insert can be verified by sequencing.

As an alternative, the dominant negative PML mutant can be generated from wild-type PML cDNA as follows. The cDNA from wild type PML can be isolated as described elsewhere (Goddard et al., 1995, Mamm Genome 6, 732-737). Subsequently, a dominant negative mutant form can be amplified from wild-type cDNA using a primer in which the G residue that represents G-962 is deleted. The coding sequence for the dominant negative form of PML can be amplified from wild-type PML cDNA using the Expand 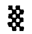 Long Template PCR system according to the standard protocol of the supplier (Boehringer Mannheim) with the following primers: 
PMLdN5A: 5'- CCC AAG CTT GCC ACC ATG CCT CCC  CCA GAG GAA CCC
PMLdN3A: 5' CGC GGA TCC GCT TCA TCC TCT TGA CCA GCG CCC CAC.
TAG TGC GGA TGC GCT GCA GCA CAG CTT CAG GCA GCT CAG CTG
 PMLdN5 contains a HindIII restriction site (bold) for easy cloning and a Kozak sequence (underlined) for optimal translation of the messenger. PMLdN3 contains a BamHI restriction site (bold) for easy cloning and a C-mutation immediately adjacent to the underlined C residue, which corresponds to the G-962 mutation as found in PML-F12dG. The amplified DNA fragment of approximately 950 bp can subsequently be digested with HindIII and BamHI and ligated into the HindIII/BamHI digested pAd5/CLIP vector, yielding pAd5/CLIP.PMLdN.

### Generation of adapter plasmid pAd5/CLIP.LacZ

The adapter plasmid pAd5/CLIP.LacZ was generated as follows: The E.coli LacZ gene was amplified from the plasmid pMLP.nlsLacZ (EP 95-202 213) by PCR with the primers 5'GGGGTGGCCAGGGTACCTCTAGGCTTTTGCAA and 5'GGGGGGATCCATAAACAAGTTCAGAATCC. The PCR reaction was performed Ex Taq (Takara) according to the suppliers protocol at the following amplification program: 5 minutes 94°C, 1 cycle; 45 seconds 94°C and 30 seconds 60°C and 2 minutes 72°C, 5 cycles; 45 seconds 94°C and 30 seconds 65°C and 2 minutes 72°C, 25 cycles; 10 minutes 72; 45 seconds 94°C and 30 seconds 60°C and 2 minutes 72°C, 5 cycles, I cycle. The PCR product was subsequently digested with Kpnl and BamHI and the digested DNA fragment was ligated into KpnI/BamHI digested pcDNA3 (Invitrogen), giving rise to pcDNA3.nlsLacZ. Next, the plasmid pAd5/CLIP was digested with SpeI. The large fragment containing part of the 5' part CMV promoter and the adenoviral sequences was isolated. The plasmid pcDNA3.nlsLacZ was digested with SpeI and the fragment containing the 3'part of the CMV promoter and the lacZ gene was isolated. Subsequently, the fragments were ligated, giving rise to pAd5/CLIP.LacZ. The reconstitution of the CMV promoter was confirmed by restriction digestion.

### Generation of adapter plasmids for the production of dual vectors expressing a dominant mutant of PML in addition to a second gene of interest.

In order to construct an adapter plasmid that allows the generation of a recombinant adenoviral vector containing both the PMLdN cDNA and a second cDNA of interest, we will use the internal ribosome entry site (IRES) of the encephalomyocarditis virus (EMCV, Jang et al., 1988; J. Virol. 62, 2636-2643). In this non-limiting example we use the cDNA coding for the E.coli β-galactosidase (LacZ) as the second gene of interest. The LacZ coding sequences can be amplified from the plasmid pAd5/CLIP.LacZ by PCR with the following primers: 
LacZ5Nco: 5'-CAT GCC ATG GAT AAA GTT TTC CGG AAT TCC GCC.
LacZ3Sal: 5'-ACG CGT CGA CTC TAG ACT TAT TTT TGA CAC CAG ACC
AAC TG.
 Primer LacZ5Nco contains a NcoI site (bold) for easy cloning. The primer LacZ3Sal contains a SalI site (bold) and XbaI site (underlined) for easy cloning. The PCR reaction will be performed according to the standard protocol of the supplier (Boehringer Mannheim) using the Expand 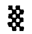 Long Template PCR system. The PCR product can subsequently be digested with NcoI and SalI. The vector pBr/PTkEMCVNeo/2 (WO 96/35798) will be digested with SalI and partially digested with NcoI to yield a vector fragment of 6866 bp that can be purified on LMP agarose gel. Next, this fragment will be ligated to the NcoI/SalI digested PCR product, yielding pBr/PTkEMCVLacZ. This plasmid can be digested with XbaI and the XbaI fragment harbouring the LacZ cDNA downstream of the EMCV IRES will be purified and cloned into XbaI site of the pAd/CLIP.PMLdN adapter plasmid. The resulting adapter plasmid pAd/CLIP.PMLdN.LacZ has the PMLdN coding sequences immediately downstream of the CMV promoter, followed by the IRES, followed by the LacZ coding sequences. Recombinant adenoviral vectors derived from the pAd/CLIP.PMLdN.LacZ introduce a bi-cistronic mRNA in the infected cells that is transcribed from the CMV promoter and facilitates the translation of both PMLdN and LacZ.

### Generation of pBr/Ad.Bam-rITR (ECACC deposit P97082122)

In order to facilitate blunt end cloning of the ITR sequences, wild-type human adenovirus type 5 (Ad5) DNA was treated with Klenow enzyme in the presence of excess dNTPs. After inactivation of the Klenow enzyme and purification by phenol/chloroform extraction followed by ethanol precipitation, the DNA was digested with BamHI. This DNA preparation was used without further purification in a ligation reaction with pBr322 derived vector DNA prepared as follows: pBr322 DNA was digested with EcoRV and BamHI, dephosphorylated by treatment with TSAP enzyme (Life Technologies) and purified on LMP agarose gel (SeaPlaque GTG). After transformation into competent *E.coli* DH5α (Life Techn.) and analysis of ampiciline resistant colonies, one clone was selected that showed a digestion pattern as expected for an insert extending from the BamHI site in Ad5 to the right ITR. Sequence analysis of the cloning border at the right ITR revealed that the most 3' G residue of the ITR was missing, the remainder of the ITR was found to be correct. Said missing G residue is complemented by the other ITR during replication.

### Generation of pBr/Ad.Sal-rITR (ECACC deposit P97082119)

pBr/Ad.Bam-rITR was digested with BamHI and SalI. The vector fragment including the adenovirus insert was isolated in LMP agarose (SeaPlaque GTG) and ligated to a 4.8 kb SalI-BamHI fragment obtained from wt Ad5 DNA and purified with the Geneclean II kit (Bio 101, Inc.). One clone was chosen and the integrity of the Ad5 sequences was determined by restriction enzyme analysis. Clone pBr/Ad.Sal-rITR contains adeno type 5 sequences from the SalI site at bp 16746 up to and including the rITR (missing the most 3' G-residue).

### pBr/Ad.Cla-Bam (ECACC deposit P97082117)

wt Adeno type 5 DNA was digested with *Cla*I and *Bam*HI, and the 20.6 kb fragment was isolated from gel by electro-elution. pBr322 was digested with the same enzymes and purified from agarose gel by Geneclean. Both fragments were ligated and transformed into competent DH5α. The resulting clone pBr/Ad.Cla-Bam was analysed by restriction enzyme digestion and shown to contain an insert with adenovirus sequences from bp 919 to 21566.

### Generation of pBr/Ad.AflII-Bam (ECACC deposit P97082114)

Clone pBr/Ad.Cla-Bam was linearised with EcoRI (in pBr322) and partially digested with AflII. After heat inactivation of AflII for 20' at 65°C, the fragment ends were filled in with Klenow enzyme. The DNA was then ligated to a blunt double stranded oligo linker containing a PacI site (5'-AATTGTCTTAATTAACCGCTTAA-3'). This linker was made by annealing the following two oligonucleotides: 5'-AATTGTCTTAATTAACCGC-3' and 5'-AATTGCGGTTAATTAAGAC-3', followed by blunting with Klenow enzyme. After precipitation of the ligated DNA to change buffer, the ligations were digested with an excess PacI enzyme to remove concatamers of the oligo. The 22016 bp partial fragment containing Ad5 sequences from bp 3534 up to 21566 and the vector sequences, was isolated in LMP agarose (SeaPlaque GTG), re-ligated and transformed into competent DH5a. One clone that was found to contain the PacI site and that had retained the large adeno fragment was selected and sequenced at the 5' end to verify correct insertion of the PacI linker in the (lost) AflII site.

### Generation of pBr/Ad.Bam-rITRpac#2 (ECACC deposit P97082120) and pBr/Ad.Bam-rITR#8 (ECACC deposit P97082121)

To allow insertion of a PacI site near the ITR of Ad5 in clone pBr/Ad.Bam-rITR, about 190 nucleotides were removed between the ClaI site in the pBr322 backbone and the start of the ITR sequences. This was done as follows: pBr/Ad.Bam-rITR was digested with ClaI and treated with nuclease Bal31 for varying lengths of time (2', 5', 10' and 15'). The extent of nucleotide removal was followed by separate reactions on pBr322 DNA (also digested at the ClaI site), using identical buffers and conditions. Bal31 enzyme was inactivated by incubation at 75°C for 10 minutes, the DNA was precipitated and re-suspended in a smaller volume TE buffer. To ensure blunt ends, DNA's were further treated with T4 DNA polymerase in the presence of excess dNTPs. After digestion of the (control) pBr322 DNA with SalI, satisfactory degradation (^{~}150 bp) was observed in the samples treated for 10' or 15'. The 10' or 15' treated pBr/Ad.Bam-rITR samples were then ligated to the above-described blunted PacI linkers (See pBr/Ad.AflII-Bam). Ligations were purified by precipitation, digested with excess PacI and separated from the linkers on an LMP agarose gel. After re-ligation, DNA's were transformed into competent DH5a and colonies analysed. Ten clones were selected that showed a deletion of approximately the desired length and these were further analysed by T-track sequencing (T7 sequencing kit, Pharmacia Biotech). Two clones were found with the PacI linker inserted just downstream of the rITR. After digestion with PacI, clone #2 has 28 bp and clone #8 has 27 bp attached to the ITR.

Generation of pWE/Ad.AflII-rITR (ECACC deposit P97082116)

Cosmid vector pWE15 (Clontech) was used to clone larger Ad5 inserts. First, a linker containing a unique PacI site was inserted in the EcoRI sites of pWE15 creating pWE.pac. To this end, the double stranded PacI oligo, as described for pBr/Ad.AflII-BamHI, was used but now with its EcoRI protruding ends. The following fragments were then isolated by electro-elution from agarose gel: pWE.pac digested with PacI, pBr/AflII-Bam digested with PacI and BamHI and pBr/Ad.Bam-rITR#2 digested with BamHI and PacI. These fragments were ligated together and packaged using λ phage packaging extracts (Stratagene) according to the manufacturer's protocol. After infection into host bacteria, colonies were grown on plates and analysed for presence of the complete insert. pWE/Ad.AflII-rITR contains all adenovirus type 5 sequences from bp 3534 (AflII site) up to and including the right ITR (missing the most 3' G residue).

### Generation of pWE/Ad.AflII-rITRΔE2A:

Deletion of the E2A coding sequences from pWE/Ad.AflII-rITR (ECACC deposit P97082116) has been accomplished as follows. The adenoviral sequences flanking the E2A coding region at the left and the right site were amplified from the plasmid pBr/Ad.Sal.rITR (ECACC deposit P97082119) in a PCR reaction with the Expand PCR system (Boehringer) according to the manufacturers protocol. The following primers were used:

### Right flanking sequences (corresponding Ad5 nucleotides 24033 to 25180):

ΔE2A.SnaBI:     5'-GGC GTA CGT AGC CCT GTC GAA AG-3'

ΔE2A.DBP-start: 5'-CCA ATG CAT TCG AAG TAC TTC CTT CTC
CTA TAG GC-3'

The amplified DNA fragment was digested with SnaBI and NsiI (NsiI site is generated in the primer ΔE2A.DBP-start, underlined).

Left flanking sequences (corresponding Ad5 nucleotides 21557 to 22442): 
ΔE2A.DBP-stop: 5'-CCA ATG CAT ACG GCG CAG ACG G-3'
ΔE2A.BamHI: 5'-GAG GTG GAT CCC ATG GAC GAG-3'
 The amplified DNA was digested with BamHI and NsiI (NsiI site is generated in the primer ΔE2A.DBP-stop, underlined). Subsequently, the digested DNA fragments were ligated into SnaBI/BamHI digested pBr/Ad.Sal-rITR. Sequencing confirmed the exact replacement of the DBP coding region with a unique NsiI site in plasmid pBr/Ad.Sal-rITRΔE2A. The unique NsiI site can be used to introduce an expression cassette for a gene to be transduced by the recombinant vector, e.g. PML or PMLdN.

Next, the plasmid pWE/Ad.AflII-rITRΔE2A was generated. The plasmid pBr/Ad.Sal-rITRΔE2A was digested with BamHI and SpeI. The 3.9-Kb fragment, in which the E2A-coding region was replaced by the unique NsiI site, was isolated. The pWE/Ad.AflII-rITR was digested with BamHI and SpeI. The 35 Kb DNA fragment, from which the BamHI/SpeI fragment containing the E2A coding sequence was removed, was isolated. The fragments were ligated and packaged using λ phage-packaging extracts according to the manufacturer protocol (Stratagene), yielding the plasmid pWE/Ad.AflII-rITRΔE2A.

### Example 2

### Generation of recombinant adenoviruses

### E1-deleted recombinant adenoviruses

To generate E1 deleted recombinant adenoviruses with the plasmid-based system, the following constructs were prepared:
a) An adapter construct containing the expression cassette with the gene(s) of interest linearised with a restriction enzyme that cuts at the 3' side of the overlapping adenoviral genome fragment, preferably removing the pBr322 vector sequences, and
b) A complementing adenoviral genome construct pWE/Ad.AflII-rITR digested with PacI.
These two DNA molecules are further purified by phenol/chloroform extraction and ethanol precipitation. Co-transfection of these plasmids into a suitable adenovirus packaging cell line generates recombinant replication deficient adenoviruses by a one-step homologous recombination between the adapter and the complementing adenoviral genome.

A general protocol as outlined below and meant as a non-limiting example of the present invention will be performed to produce several recombinant adenoviruses using various adapter plasmids and the Ad.AflII-rITR fragment. Adenovirus packaging cells are seeded in ^{~}25 cm² flasks and the next day, when they are at ^{~}80% confluency, transfected with a mixture of DNA and lipofectamine agent (Life Techn.) as described by the manufacturer. Routinely, 40µl lipofectamine, 4µg adapter plasmid and 4 µg of the complementing adenovirus genome fragment AflII- rITR are used. Under these conditions transient transfection efficiencies of ^{~}50% (48 hrs post transfection) are obtained as determined with control transfections using a pAd5/CLIP.LacZ adapter. Two days later, cells will be passaged to ^{~}80 cm² flasks and further cultured. Approximately five (for the single homologous recombination) to eleven days (for the double homologous recombination) later a cytopathic effect (CPE) is expected, indicating that functional adenovirus has formed. Cells and medium are harvested upon full CPE and recombinant virus is released by freeze-thawing. An extra amplification step in a 80 cm² flask is routinely performed to increase the yield, since at the initial stage the titers are found to be variable despite the occurrence of full CPE. After amplification, viruses will be harvested and plaque purified on PER.C6 cells. Individual plaques will be tested for viruses with active trans-genes.

Several different recombinant adenoviruses, comprising the LacZ gene (IG.Ad.CLIP.LacZ), the PMLdN gene (IG.Ad.CLIP.PMLdN) or a cassette for both PMLdN and LacZ (IG.Ad.CLIP.PMLdN.LacZ) will be produced using this protocol.

### Generation of recombinant adenoviruses deleted for early region 1 and early region 2A

Besides replacements in the E1 region, it is possible to delete or replace the E2A region in the adenovirus. This creates the opportunity to use a larger insert or to insert more than one gene without exceeding the maximum packagable size (approximately 105% of wt genome length).

Recombinant viruses that are both E1 and E2A deleted are generated by a homologous recombination procedure as described above for E1-replacement vectors using a plasmid-based system consisting of:
a) An adapter plasmid for El replacement according to the invention.
b) The pWE/Ad.AflII-rITRΔE2A fragment, with or without insertion of a second gene of interest.
Generation and propagation of such viruses, e.g. IG.Ad.CLIP.PMLdNΔE2A, IG.Ad.CLIP.LacZΔE2A and IG.Ad.CLIP.PMLdN.LacZΔE2A requires a complementing cell line for complementation of both the E1 and E2A proteins in trans.

In addition to replacements in the E1 and E2A region, it is also possible to delete or replace (part of) the E3 region in the E1- deleted adenoviral vector, because E3 functions are not necessary for the replication, packaging and infection of the (recombinant) virus. This creates the opportunity to use larger inserts or to insert more than one gene without exceeding the maximum packagable size (approximately 105% of wt genome length). This can be done, e.g., by deleting part of the E3 region in the pBr/Ad.Bam-rITR clone by digestion with XbaI and re-ligation. This removes Ad5 wt sequences 28592-30470 including all known E3 coding regions. Another example is the precise replacement of the coding region of gpl9K in the E3 region with a polylinker allowing insertion of new sequences. This, 1) leaves all other coding regions intact and 2) obviates the need for a heterologous promoter since the transgene is driven by the E3 promoter and pA sequences, leaving more space for coding sequences.

To this end, the 2.7-kb EcoRI fragment from wt Ad5 containing the 5' part of the E3 region was cloned into the EcoRI site of pBluescript (KS⁻) (Stratagene). Next, the HindIII site in the polylinker was removed by digestion with EcoRV and HincII and subsequent re-ligation. The resulting clone pBS.Eco-Eco/ad5ΔHIII was used to delete the gp19K-coding region. Primers 1 (5'-GGG TAT TAG GCC AA AGG CGC A-3') and 2 (5'-GAT CCC ATG GAA GCT TGG GTG GCG ACC CCA GCG-3') were used to amplify a sequence from pBS.Eco-Eco/ad5ΔHIII corresponding to sequences 28511 to 28734 in wt Ad5 DNA. Primers 3 (5'-GAT CCC ATG GGG ATC CTT TAC TAA GTT ACA AAG CTA-3') and 4 (5'-GTC GCT GTA GTT GGA CTG G-3') were used on the same DNA to amplify Ad5 sequences from 29217 to 29476. The two resulting PCR fragments were ligated together by virtue of the new introduced NcoI site and subsequently digested with XbaI and MunI. This fragment was then ligated into the pBS.Eco-Eco/ad5ΔHIII vector that was digested with XbaI (partially) and MunI generating pBS.Eco- . Eco/ad5ΔHIII.Δgp19K. To allow insertion of foreign genes into the HindIII and BamHI site, an XbaI deletion was made in pBS.Eco-Eco/adSΔHIII.Δgp19K to remove the BamHI site in the Bluescript polylinker. The resulting plasmid pBS.Eco-Eco/ad5ΔHIIIΔgp19KΔxbaI, contains unique HindIII and BamHI sites corresponding to sequences 28733 (HindIII) and 29218 (BamHI) in Ad5. After introduction of a foreign gene, e.g. HindIII/BamHI digested PMLdN, into these sites, either the deleted XbaI fragment is re-introduced, or the insert is recloned into pBS.Eco-Eco/adSΔHIII.Δgp19K using HindIII and for example MunI. Using this procedure, we have generated plasmids expressing HSV-TK, hIL-1α, rat IL-3, luciferase or LacZ, but also PMLdN expression plasmids can be generated in this way. The unique SrfI and NotI sites in the pBS.Eco-Eco/ad5ΔHIII.Δgp19K plasmid (with or without inserted gene of interest) are used to transfer the region comprising the gene of interest into the corresponding region of pBr/Ad.Bam-rITR, yielding construct pBr/Ad.Bam-rITRΔgp19K (with or without inserted gene of interest). This construct is used as described *supra* to produce recombinant adenoviruses. In the viral context, expression of inserted genes is driven by the adenovirus E3 promoter.

Recombinant viruses that are both E1 and E3 deleted are generated by a double homologous recombination procedure for E1-replacement vectors using a plasmid-based system consisting of:
a) an adapter plasmid for E1 replacement according to the invention, with or without insertion of a first gene of interest,
b) the pWE/Ad.AflII-EcoRI fragment, and
c) the pBr/Ad.Bam-rITRΔgp19K plasmid with or without insertion of a second gene of interest.

In addition to manipulations in the E3 region, changes of (parts of) the E4 region can be accomplished easily in pBr/Ad.Bam-rITR. Moreover, combinations of manipulations in the E3 and/or E2A and/or E4 region can be made. Generation and propagation of such vectors, however, demands packaging cell lines that complement for E2A and/or E4 *in trans.*

### Example 3

### Down-regulation of cell surface expression of MHC-class I by recombinant adenoviral vectors expressing dominant negative PML.

In order to study whether dominant negative mutant PML is able to down-regulate the expression of MHC class I molecules on the cell surface on recombinant adenovirus infected cells, the following experiment can be performed: HeLa cells (ATCC CCL-2) or Balb/c 3T3 cells (ATCC CCL-163, MHC haplotype d) were seeded at 1x10⁶ cells per tissue culture plate (Greiner) in Dulbecco's Modified Eagle Medium (DMEM, Gibco BRL) supplemented with 10% Fetal Bovine Serum (FBS, Gibco BRL) in a 10% CO₂ atmosphere at 37°C. The next day, cells were infected with a multiplicity of infection (m.o.i.) of either 0, 10, 100 or 1000 viral particles per cell IG.Ad/CMV.LacZ, IG.Ad/CMV.LacZΔE2A, IG.Ad.CLIP.PMLdN, IG.Ad.CLIP.PMLdNΔE2A, IG.Ad.CLIP.PMLdN.LacZ or IG.Ad.CLIP.PMLdN.LacZΔE2A per cell. Forty-eight hours post infection, cells were either assayed for MHC class I cell surface expression or LacZ expression.

Cell surface expression of MHC class I antigens was determined as follows: Infected cells are washed once with PBS, trypsinised, washed twice with PBS containing 2 % FCS (FACS buffer) and adjusted to 2x10⁷/ml in FACS buffer. 20 µl of 100 fold in FACS buffer diluted anti class I antibody is added to 50 µl of cell suspension in v-shaped microtiter wells. The monoclonal anti-human class I antibody specific for correctly folded HLA-A, B and C locus heavy chains associated with β₂m as described by Barnstable et al. will be used for detection of MHC class I molecules on HeLa cells (Barnstable *et al.*, 1977, Cell 14:9-20). MHC class I molecules on infected 3T3 cells can be detected by mouse monoclonal antibodies directed against mouse H2-D^{d} (clone 34-5-8S, PharMingen), H2-K^{d} (clone SF1-1.1, PharMingen) and H2-L^{d} ( clone 28-14-8, PharMingen). The mixtures are incubated on ice for 30-45min. The plate is centrifuged at 200 x g for 3 min. at 4°C and supernatants are removed. Cell pellets are washed twice in 100 µl FACS buffer and re-suspended in 25 µl FACS buffer. To each well 5 µl Goat anti-mouse IgG-FITC labelled antibody (Becton Dickinson, Catalog No.9031) is added and plates are incubated for an additional 30-45 min. on ice. Cells are centrifuged 200 x g, washed 2 times in cold FACS buffer and re-suspended at 2 x10⁶/ml in cold FACS buffer. Cell surface expression of MHC class I molecules is analysed using the FACS scan (FACSort, Becton Dickinson). Cells infected with either IG.Ad.CLIP.PMLdN, IG.Ad.CLIP.PMLdNΔE2A, IG.Ad.CLIP.PMLdN.LacZ or IG.Ad.CLIP.PMLdN.LacZΔE2A expressed reduced levels of MHC class I molecules at their cell surface as compared to IG.Ad/CMV.LacZ or IG.Ad/CMV.LacZΔE2A infected cells. These data indicate that expression of PMLdN reduces the cell surface expression of MHC class I molecules on the recombinant adenovirus infected cells.

The LacZ transducing efficiency is determined as follows: Infected cells are washed twice with PBS (NPBI) and fixed for 8 minutes in 0.25% glutaraldehyde (Sigma) in PBS (NPBI). Subsequently, the cells were washed twice with PBS and stained for 8 hours with X-gal solution (1 mg/ml X-gal in DMSO (Gibco), 2mM MgCl₂ (Merck), 5mM K₄[Fe(CN)₆].3H₂O (Merck), 5mM K₃[Fe(CN)₆] (Merck) in PBS. The reaction was stopped by removal of the X-gal solution and washing of the cells with PBS. Cells infected with either IG.Ad/CMV.LacZ, IG.Ad/CMV.LacZΔE2A, IG.Ad.CLIP.PMLdN.LacZ or IG.Ad.CLIP.PMLdN.LacZΔE2A expressed the LacZ gene as evidenced by blue staining of the cells. The transducing efficiency increases with increasing m.o.i.'s. The cells that are infected with either IG.Ad.CLIP.PMLdN or IG.Ad.CLIP.PMLdNΔE2A do not express the LacZ gene. Taken together, these data indicate that expression of PMLdN reduces the cell surface expression of MHC class I molecules on the recombinant adenovirus infected cells, without interfering with the expression of a second gene that is carried by the same recombinant adenoviral vector.

### Example 4

### Longevity of expression of a transgene from recombinant adenoviral vectors expressing a dominant negative mutant of PML

### Increased duration of expression of a reporter gene from dual recombinant adenoviral vectors that co-expresses PMLdN.

In order to study whether PMLdN mediated down-regulation of MHC class I cell surface expression increases the longevity of expression of transgene from recombinant adenoviral vectors *in vivo,* the following experiments are performed. A total of 10⁸ or 10⁹ virus particles of either IG.Ad/CMV.LacZ, IG.Ad/CMV.LacZΔE2A, IG.Ad.CLIP.PMLdN.LacZ or IG.Ad.CLIP.PMLdN.LacZΔE2A are injected into the tail vein of 8 weeks old C57/B16 or NOD-SCID mice. At day 7, 14, 28, and 56, two mice per group are sacrificed, the livers of these mice are isolated and fixed in formalin. Thin slices were cut and washed extensively in PBS. Subsequently, the slices were stained in X-gal solution (1 mg/ml X-gal in DMSO (Gibco), 2mM MgCl₂ (Merck), 5mM K₄[Fe(CN)₆].3H₂O (Merck), 5mM K₃[Fe(CN)₆] (Merck) in PBS. After an 8-hour incubation, the samples are washed in PBS. The longevity of LacZ expression from dual adenoviral vectors expressing both PMLdN and LacZ is increased as compared to LacZ expression from recombinant adenoviral vectors that only express LacZ in immuno-competent C57/B16 mice. In contrast, the LacZ expression in the livers of immuno-deficient NOD-SCID mice is stable in all cases. These data indicate that PMLdN down-regulates the cell surface expression of MHC class I molecules of the adenovirus transduced cells *in vivo.* As a result, these cells render 'invisible' for the cytotoxic T-cells and therefore escape T-cell mediated eradication of the adenovirus transduced cells from the animal. This finally results in an increase in duration of the expression of the gene of interest.

### CITED LITERATURE

1. Bout,A., Pharmaceutical Biotechnology, 167 (1997)
2. Ploegh, H.L. Science 280, 247 (1998)
3. Goldberg,A.L. and K.L. Rock, Nature, 357, 375 (1992).
4. Ciechanover, A., Cell, 79, 13 (1994).
5. Groettrup, M. *et al.*, Immunol. Today, 17, 429 (1996).
6. Bertoletti, A. *et al.*, Nature, 369, 407 (1994).
7. Klenerman, P. *et al.*Nature, 369, 403 (1994).
8. De Campos-Lima,P.O. *et al.* J.Exp.Med. 179, 1297 (1994).
9. Gilbert, M.J. *et al.,* Nature, 383, 720 (1996).
10. Levitskaya, J. *et al.* Proc. Natl. Acad. Sci. USA. 94, 12616 (1997).
11. Hill, A. *et al.*, Nature, 375, 411 (1995).
12. York, I.A. *et al.*, Cell, 77, 525 (1994).
13. Hengel, H. *et al.*Immunity, 6, 623 (1997).
14. Ahn, K. *et al.*, Immunity, 6, 3247 (1997).
15. Paabo, S. *et al.*, Adv. Cancer Res., 52, 151 (1989).
16. Ahn, K. *et al.*, Proc. Natl. Acad. Sci. USA 93, 10990 (1996).
17. Jones, T.R. *et al.,* Proc. Natl. Acad. Sci. USA, 93, 11327 (1996).
18. Ziegler, H., *et al.*, Immunity, 6, 57 (1997).
19. Wiertz, E.J., *et al.,* Nature, 384, 432 (1996).
20. Hengel, H. and U.H. Koszinowski, Curr. Opin. Immunol., 9, 407 (1997).
21. Kerkau, T. *et al.*, J. Exp. Med., 185, 195 (1997).
22. Schwartz, O., *et al.,* Nature Med., 2, 338 (1996).
23. Greenberg, M.E., *et al.,* EMBO. J., 16, 6964 (1997).
24. Collins, K.L., *et al.,* Nature, 391, 397 (1998).
25. Kleijnen, M.F., *et al.,* EMBO. J., 16, 685 (1997).
26. Borrow, J. et *al.,* Science, 249, 1577 (1990).
27. Longo, L. *et al.,* J.Exp.Med., 172, 1571 (1990).
28. De The, H., *et al.,* Nature, 347, 558 (1990).
29. De The, H., *et al.,* Cell, 66, 675 (1991).
30. Kakizuka, A., *et al.,* Cell, 66, 663 (1991).
31. Goddard, A.D., *et al.,* Science, 254, 1371 (1991).
32. Goddard, A.D., *et al.,* Mamm Genome, 6, 732 (1995).
33. Fagioli, M., *et al.,* Oncogene, 7, 1083 (1992).
34. Borden, K.L.B., *et al.,* EMBO J., 14, 1532 (1995).
35. Freeman, P.S., *et al.*, Cell, 64, 483 (1991).
36. Freeman, P.S., Ann. NY Acad. Sci., 684, 174 (1993).
37. Kastner, P., *et al.,* EMBO J., 11, 629 (1992).
38. Perez, A., *et al.,* EMBO J., 12, 3171 (1993).
39. Doucas, V., *et al.*, Biochim. Biophys. Acta, 1288, M25 (1996).
40. Zheng, P, *et al.,* Nature, 396, 373 (1998)
41. Otake, K, *et al.*, Hum Gene Ther. 10, 2207 (1998).
42. Lieber, A, *et al.*, J Virol. 72 , 9267 (1998).
43. Benhamou PY, *et al.*, Diabetologia. 41, 1093 (1998).
44. Zhang, HG, *et al.*, Hum Gene Ther 9, 1875 (1998).
45. Yang Y, *et al.*, J Virol 70 ,7209 (1996)
46. Wilson CB, et al., J Virol 1998 72 7542 (1998).
47. Jooss K, *et al.*, Gene Ther 5, 309 (1998).
48. Yang Y, *et al.*, Hum Mol Genet 5 ,1703 (1996).
49. Zsengeller ZK, *et al.*, Hum Gene Ther 6, 457 (1995).

## Claims

1. A method for at least in part inhibiting an immune response against specific cells in a body without essentially affecting the capacity of the immune system in said body to mount other immune responses, comprising providing said cells with a nucleic acid delivery vehicle comprising at least one nucleic acid sequence encoding at least one protein capable of at least in part inhibiting peptide presentation by major histocompatibility complex I.

2. A method according to claim 1 wherein said protein exhibits two or more activities inhibiting peptide presentation by major histocompatibility complex I.

3. A method according to claim 1 or claim 2, wherein at least part of said protein is derived from a protein naturally present in a cell, preferably a eukaryotic cell.

4. A method according to anyone of claims 1-3, wherein at least part of said protein is derived from a protein naturally present in a mammalian cell.

5. A method according to anyone of claims 1-4, wherein at least part of said protein is derived from a protein naturally present in a human cell.

6. A method according to anyone of claims 1-5, wherein said protein comprises a mutant PML or a functional part, derivative or analogue thereof.

7. A method according to claim 6, wherein said mutant PML comprises an amino-acid sequence of PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L) or functional part, derivative or analogue thereof.

8. A method according to anyone of claims 1-7, wherein said cells comprise cells of a transplant.

9. A method according to anyone of claims 1-8, wherein said cells comprise target cells of an auto-immune response.

10. A method according to anyone of claims 1-9, wherein said cells comprise pancreatic islet β-cells or Langerhans cells.

11. A method according to anyone of claims 1-10, wherein said cells are further provided with a nucleic acid encoding at least one protein with a different activity, preferably a therapeutic activity.

12. A method according to claim 11, wherein said protein with a different activity is a nitric oxide synthetase or a part, derivative or functional analogue thereof.

13. A method according to claim 11, wherein said protein is a secreted protein, preferably an interleukin-3 or a functional part, derivative or analogue thereof.

14. A method according to anyone of claims 1-13, wherein said cells are further provided with a nucleic acid encoding at least one additional protein capable of providing cells with the capacity to at least in part evade an immune response.

15. A method according to anyone of claims 1-14, wherein the capacity of the immune system in said body, to mount an immune response against said cells in said body, is additionally at least in part inhibited through administering to said body at least one compound capable of inhibiting immune responses in an essentially non-specific way.

16. A nucleic acid delivery vehicle comprising a nucleic acid sequence encoding at least one protein capable of at least in part inhibiting peptide presentation by major histocompatibility complex I.

17. A nucleic acid delivery vehicle according to claim 16 wherein said protein exhibits two or more activities inhibiting peptide presentation by major histocompatibility complex I.

18. A nucleic acid delivery vehicle according to claim 16 or claim 17, wherein at least part of said protein is derived from a protein naturally present in a cell, preferably a eukaryotic cell.

19. A nucleic acid delivery vehicle according to anyone of claims 16-18, wherein at least part of said protein is derived from a protein naturally present in a mammalian cell.

20. A nucleic acid delivery vehicle according to anyone of claims 16-19, wherein at least part of said protein is derived from a human cell.

21. A nucleic acid delivery vehicle according to anyone of claims 16-20, wherein said protein comprises a mutant PML or a functional part, derivative or analogue thereof.

22. A nucleic acid delivery vehicle according to claim 21, wherein said mutant PML comprises an amino-acid sequence of PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L), or functional part, derivative or analogue thereof.

23. A nucleic acid delivery vehicle according to anyone of claims 16-22, wherein expression of said protein is under control of a promoter of which the activity can be regulated.

24. A nucleic acid delivery vehicle according to anyone of claims 16-23, further comprising a nucleic acid encoding at least one protein with a different activity, preferably a therapeutic activity.

25. A nucleic acid delivery vehicle according to claim 24, wherein said protein with a different activity is a nitric oxide synthetase or a part, derivative or functional analogue thereof.

26. A nucleic acid delivery vehicle according to claim 24, wherein said protein with a different activity is a secreted protein, preferably interleukin-3 or a part, derivative or functional analogue thereof.

27. A nucleic acid delivery vehicle according to anyone of claims 16-26, further provided with a nucleic acid sequence encoding a herpes simplex virus thymidine kinase or a part, derivative or functional analogue thereof.

28. A nucleic acid delivery vehicle according to anyone of claims 16-27, further comprising a nucleic acid sequence encoding at least one additional protein capable of providing cells with the capacity to at least in part evade an immune response.

29. A nucleic acid delivery vehicle according to anyone of claims 16-28, wherein said vehicle has at least in part been provided with a tissue tropism for smooth muscle cells and/or endothelial cells.

30. A nucleic acid delivery vehicle according to anyone of claims 16-29, wherein said vehicle has at least in part been deprived of tissue tropism for liver cells.

31. A nucleic acid delivery vehicle according to claim 29 or claim 30, wherein said changed tissue tropism is provided by at least a tissue tropism determining part of a fiber protein of a group B adenovirus, preferably adenovirus 16.

32. A nucleic acid delivery vehicle according to anyone of claims 16-31, wherein said nucleic acid further comprises integration means of an adeno-associated virus nucleic acid.

33. A nucleic acid delivery vehicle according to anyone of claims 16-32, wherein said vehicle is an adenovirus particle, an adeno-associated virus particle or a retrovirus particle.

34. An adenovirus particle according to claim 33, wherein said nucleic acid further comprises an adenovirus vector and wherein said vector is at least in part deprived of the capability to express E1-region encoded proteins, preferably through a deletion in the E1-region.

35. An adenovirus particle according to claim 34, wherein said vector is further at least in part deprived of the capability to express E2A protein.

36. An adenovirus particle according to claim 34 or claim 35 wherein said vector is further at least in part deprived of the capability to express at least one of the E3-region encoded proteins.

37. An adenovirus particle according to anyone of claims 34-36, wherein said vector is further at least in part deprived of the capability to express at least one of the E4-region encoded proteins, preferably E4-Orf3 and/or E4-Orf6.

38. A cell for the production of an adenovirus particle according to claim 34, claim 35 or claim 37, comprising one or more means for the complementation of at least part of the function of one or more of the proteins said vector has at least in part been deprived the capability of expression of.

39. A cell according to claim 38, wherein one of said means comprises a mutant PML or a functional part, derivative or analogue thereof.

40. A cell according to anyone of claim 38 or claim 39, wherein said cell is derived from a PER.C6 cell (ECACC deposit number 96022940).

41. Use of a protein capable of inhibiting peptide presentation by major histocompatibility complex I, for at least in part preventing immune response related removal of a selected population of cells from a body without essentially affecting the capacity of the immune system in said body to mount other immune responses.

42. A use according to claim 41, wherein said protein exhibits two or more activities inhibiting said peptide presentation.

43. A use according to claim 41 or claim 42, wherein said protein is derived from a protein naturally present in a cell.

44. A use according to anyone of claims 41-43, wherein said protein is a mutant PML, preferably comprising an amino-acid sequence of PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L), or functional part, derivative or analogue thereof.

45. Use of a mutant PML, preferably comprising an amino-acid sequence of PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L) or functional part, derivative or analogue thereof, in a medicament.

46. A functionally analogues protein to PML-mutant F12dG, PML.S1.H4 (figure 1B), PML/S2.H4 (figure 1D), PML/S1.H2 (figure 1F), PML/S2.H2 (figure 1H), PML/S1.dN (figure 1J) or PML/S2.dN (figure 1L) derived from a primate.

47. A nucleotide sequence encoding a protein according to claim 46, preferably such as PML.S1.H4 (figure 1A), PML/S2.H4 (figure 1C), PML/S1.H2 (figure 1E), PML/S2.H2 (figure 1G), PML/S1.dN (figure 1I) or PML/S2.dN (figure 1K) or a functional part, derivative or analogue thereof.

48. An adenovirus vector having been provided with a nucleic acid sequence encoding a protein capable of at least in part inhibiting peptide presentation by major histocompatibility complex 1.

49. An adeno-associated virus vector comprising a nucleic acid sequence encoding a protein capable of at least in part inhibiting peptide presentation by major histocompatibility complex 1.

50. A retroviral vector comprising or having been provided with a nucleic acid sequence encoding a protein capable of at least in part inhibiting peptide presentation by major histocompatibility complex 1.

51. A vector according to anyone of claims 48-50, wherein said protein comprises a mutant PML or functional part, derivative or analogue thereof.

52. A primate cell comprising a mutant PML or a functional part, derivative or analogue thereof.
